(19) **Europäisches Patentamt / European Patent Office / Office européen des brevets**

(11) **EP 3 016 696 B1**

(12) **EUROPÄISCHE PATENTSCHRIFT**

(45) Veröffentlichungstag und Bekanntmachung des Hinweises auf die Patenterteilung:
**09.01.2019   Patentblatt 2019/02**

(21) Anmeldenummer: **14736360.0**

(22) Anmeldetag: **02.07.2014**

(51) Int Cl.:
*A61M 1/34* (2006.01)          *A61M 1/36* (2006.01)
*A61M 5/142* (2006.01)        *A61M 5/168* (2006.01)
*B01F 15/02* (2006.01)

(86) Internationale Anmeldenummer:
**PCT/EP2014/064020**

(87) Internationale Veröffentlichungsnummer:
**WO 2015/000934 (08.01.2015 Gazette 2015/01)**

(54) **VORRICHTUNG ZUR TURBULENZERZEUGUNG DURCH PULSIERENDEN FLUSS**

DEVICE FOR GENERATING TURBULENCE BY PULSING FLOW

DISPOSITIF PERMETTANT DE GÉNÉRER UNE TURBULENCE PAR UN ÉCOULEMENT PULSÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.07.2013   DE 102013011010**

(43) Veröffentlichungstag der Anmeldung:
**11.05.2016   Patentblatt 2016/19**

(73) Patentinhaber: **Fresenius Medical Care Deutschland GmbH**
**61352 Bad Homburg (DE)**

(72) Erfinder: **PETERS, Arne**
**61352 Bad Homburg (DE)**

(74) Vertreter: **Schön, Andrea**
**Fresenius Medical Care AG & Co. KGaA**
**Global Intellectual Property**
**Frankfurter Straße 6-8**
**66606 St. Wendel (DE)**

(56) Entgegenhaltungen:
**WO-A1-03/095982          WO-A2-2010/029401**
**DE-A1- 10 205 056        US-A1- 2007 062 861**
**US-B1- 8 123 396**

## Beschreibung

### Technisches Gebiet

Die Erfindung betrifft die Verwendung von gepulsten Flussprofilen bei der Zugabe von Lösungen zum extrakorporalen Kreislauf, um die Durchmischung der zugegebenen Lösung mit dem Blut zu verbessern, insbesondere bei der regionalen Antikoagulation mit Citrat.

### Stand der Technik

In der WO 2009/030973 wird ein Blutschlauchsystem mit einer Infusionsstelle beschrieben, die eine Verengung aufweist. Diese Verengung dient zur Erzeugung einer Turbulenz in dieser Infusionsstelle. In der US 8,123,396 wird die Zugabe einer Flüssigkeit aus einem Reservoir zu einem Fluidstrom in Form von individuellen Tropfen beschrieben.

In der WO 2010/029401 A1, der US 2007/0062861 und WO 2012/104072 A1 werden Verfahren zum Steuern einer Blutbehandlungsmaschine für die Durchführung einer regionalen Antikoagulation mit Citronensäure beschrieben.

### Problemstellung

Bei der extrakorporalen Blutbehandlung werden Infusionslösungen oder Medikamente meist über den extrakorporalen Blutkreislauf, d.h. das verwendete Schlauchsystem, infundiert. Je nach Art der Infusionslösung kann eine schnelle Mischung der Infusionslösung mit dem Blut wünschenswert sein.

So werden bei der extrakorporalen Blutbehandlung standardmäßig Infusionslösungen zur Hemmung der Blutgerinnung infundiert, um einem möglichen Verschluss des Blutschlauchsystems vorzubeugen.

Dazu werden hauptsächlich zwei Verfahren angewendet, die systemische und die regionale Antikoagulation. Bei der regionalen Antikoagulation wird als Antikoagulanz meist eine Citratlösung verwendet, die Calcium komplexiert und so die Gerinnung des Blutes unterdrückt. Vor Rückgabe des Blutes an den Patienten muss die physiologische Konzentration an freiem Calcium wiederhergestellt werden, da zu niedrige Calciumkonzentrationen zu Nebenwirkungen führen.Deshalb wird bei der regionalen Antikoagulation dem Blut vor Reinfusion in den Patienteneine calciumhaltige Lösung zudosiert,durch die die physiologische Calciumkonzentration und damit gleichzeitig die Gerinnungsfähigkeit im Blut wiederhergestellt werden.

Die Infusion von Infusionslösungen oder Medikamenten in das Schlauchsystem des extrakorporalen Blutkreislaufs erfolgt üblicherweise über Zugabestellen in T-Form, sogenannte T-Stücke. In diesen T-Stücken herrschen aufgrund des kreisförmigen Querschnitts und der glatten Innenwandung an der Zugabestelle laminare Strömungsbedingungen vor. Zusätzlich sind die Flussraten der infundierten Lösungen im Vergleich zum Blutfluss gering.

Die weitgehend laminaren Strömungsbedingungen und die geringen Flüsse der in den Blutstrom einfließenden Infusionslösungen oder Medikamente können eine schlechte Durchmischung des Blutes mit der zugegebenen Infusionslösung bedingen. Diese mangelhafte Vermischung der beiden Flüssigkeiten an der Zugabestelle ist speziell bei der Zugabe der Calciumlösung zu Blut unerwünscht und kann vereinzelt zur Gerinnselbildung flussabwärts führen, die wiederum unter ungünstigen Umständen zur einem Verschluss des extrakorporalen Blutkreislaufs führen kann.

Um dies zu verhindern, ist die rasche und homogene Durchmischung der zugegebenen Calciumlösung mit dem Blut wünschenswert.

Das Problem kann sich aber auch bei der Zumischung anderer Flüssigkeiten ergeben, z.B. Medikamenten, wo eine schnelle Durchmischung vorteilhaft sein kann.

Im Stand der Technik ist dieses Problem bekannt und zur Lösung werden spezielle Zugabestellen, die Mittel zur Turbulenzerzeugung enthalten, beschrieben.

Die Aufgabe der vorliegenden Erfindung ist die Bereitstellung einer Vorrichtung, die auch bei Verwendung eines standardmäßigen Blutschlauchsystems mit standardmäßigen T-Stücken ohne spezielle Mittel zur Turbulenzerzeugung eine rasche und homogene Durchmischung der zwei im T-Stück zusammengeführten Flüssigkeiten gewährleisten.

Nach der Lehre der vorliegenden Erfindung wird diese Aufgabe gelöst durch eine Vorrichtung nach Anspruch 1. Besondere Ausführungsformen sind Gegenstand der abhängigen Ansprüche 2 bis 5.

### Zusammenfassung der Erfindung

Es wird ein Verfahren zum Mischen von Blut mit einer Infusionslösung A in einem T-Stück eines Schlauchsystems für einen extrakorporalen Blutkreislauf beschrieben. In dem Schlauchsystem wird das Blut von einer ersten Pumpe in der Hauptleitung gefördert und die Infusionslösung A wird von einer zweiten Pumpe aus einer Zweigleitung über das T-Stück in die Hauptleitung gefördert. Dabei wird bei dem Verfahren die zweite Pumpe von der Kontrolleinheit so gesteuert oder geregelt, dass die zweite Pumpe die Infusionslösung A mit einem gepulsten Flussprofil fördert. Das gepulste Flussprofil wird gezielt durch die Steuersignale des Kontrolleinheit erzeugt.

Bei einem gepulsten Flussprofil wechseln sich Phasen, in denen die Flüssigkeit oder die Infusionslösung gefördert wird (Flusspulse oder Pulse) und Phasen, in denen keine oder deutlich weniger Flüssigkeit gefördert wird (Flusspausen), periodisch ab und ergeben so ein sogenanntes gepulstes Flussprofil. In anderen Worten, die zweite Pumpe läuft diskontinuierlich, was bedeutet, dass in der Förderrate der zweiten Pumpe prägnante Änderungen auftreten, die Pumpe wird nicht unbedingt in periodischen Abständen gestoppt.

Das gepulste Flussprofil kann dabei ein Rechteck-Profil aufweisen, ebenso geeignet ist aber auch ein sinusförmiges Profil, ein nadelförmiges Profil oder ähnliche.

Im Fall eines Rechteckprofils wird das gepulste Flussprofil definiert über die Pulsweite (Dauer der Pumpenbetriebs oder der Förderung der Flüssigkeit) Tau2, die Pulspause (Dauer des Pumpenstillstandes) Tau1, die sich daraus ergebende Pulsfrequenz $f$ und der im Puls geförderte Flüssigkeitsmenge pro Zeit oder der Förderrate, den Pulsfluss (Vopt). Es ist auch denkbar, dass in der Pulspause die Pumpe nicht zum Stillstand kommt, sondern mit einer deutlich geringeren Förderrate weiterläuft. Dies müsste dann bei der Berechnung der mittleren Förderrate berücksichtigt werden. Aus den vorgenannten Parametern ergibt sich der mittlere Fluss oder die mittlere Förderrate (Vm) der Flüssigkeit oder der Infusionslösung A über ein längeres Zeitintervall.

Bei dem Verfahren weist der extrakorporale Blutkreislauf neben der blutführenden Hauptleitung eine oder mehrere Zweigleitungen auf, die über T-Stücke mit dem Blutkreislauf verbunden sein können. T-Stücke sind Kunststoffteile mit drei Öffnungen, die über eine T-förmige Fluidleitung verbundenen sind. Die Fluidleitung kann auch y-förmig oder ähnlich ausgebildet sein. T-Stücke können als Einsatzstück in ein Schlauchsystem eingeklebt werden, wodurch die Hauptleitung des Schlauchsystems, z.B. der blutführenden Leitung, mit einer Zweigleitung verbunden werden kann. Über diese Zweigleitungen können dem Blut im extrakorporalen Kreislauf Infusionslösungen infundiert werden.

In einer Ausführungsform des Verfahrens kann die Kontrolleinheit die erste Pumpe so steuern oder regeln, dass diese kontinuierlich läuft um das Blut mit einem stetigen Fluss zu fördern.

Für den Fachmann selbstverständlich ist, dass während einer Behandlung durch äußere Umstände Flussänderungen auftreten oder notwendig werden können, die aber die prinzipielle Funktionsweise des Verfahrens nicht beeinträchtigen.

Für den Fachmann selbstverständlich ist ebenso, dass, bedingt durch die Struktur der Pumpen, z.B. okkludierenden Pumpen, Druck- und Flusspulse entstehen können. Dieses von der Pumpenstruktur automatisch erzeugte Flussprofil ist von der Erfindung nicht umfasst.

In einer Ausführungsform des Verfahrens kann von einer dritten Pumpe zumindest eine weitere Infusionslösung B aus einer weiteren Zweigleitung in die Hauptleitung des Schlauchsystems gefördert werden.

Das Verfahren und die erfindungsgemäße Vorrichtung sind vorteilhaft, wenn eine besonders effektive Mischung von zwei Lösungen in einem Schlauchsystem erreicht werden soll.

Bei der extrakorporalen Blutbehandlung ist der Fluss der in das Blut infundierten Lösung (Infusionslösung oder Medikament) bestimmt durch die gewünschte Dosierung.

Die Dosis einer bei der extrakorporalen Blutbehandlung verwendeten Substanz kann durch den behandelnden Arzt festgelegt werden. In der Kontrolleinheit der Behandlungsmaschine können aber auch Vorschläge für die Dosierung hinterlegt sein.

Die Dosierung kann bei der extrakorporalen Blutbehandlung durch ein bestimmtes Verhältnis des Blutflusses zum Fluss (Vm) der Infusionslösung festgelegt werden. Eine Verbesserung der Durchmischung durch Erhöhung des stetigen Flusses ist nicht möglich, da dann die Dosierung nicht mehr stimmen würde.

Durch die Anwendung eines gepulsten Flussprofils kann bei einer festgelegten Dosierung, definiert durch den mittleren Fluss (Vm), der Pulsfluss (Vopt) von dem mittleren Fluss (Vm) entkoppelt werden, da sich dieser aus Pulsfluss (Vopt) und/oder Pulsweite (Tau2) und/oder Pulsfrequenz $f$ ergibt und so bei einem für die Mischung optimalen Fluss (Vopt) über die Pulsweite (Tau2) und/oder Pulsfrequenz $f$ eingestellt werden kann.

Beim Rechteckprofil, bei dem in der Pulspause keine Flüssigkeit gefördert wird, ergibt sich z.B.

$$(Vm)= f * Tau2 *\ Vopt \qquad\qquad (1)$$

Das Verfahren und die erfindungsgemäße Vorrichtung sind besonders vorteilhaft, wenn sie bei der regionalen Antikoagulation angewendet werden, um eine besonders effektive Mischung des Blutes mit den Lösungen zur regionalen Antikoagulation zu erreichen.

Ein gängiges Verfahren ist die regionale Antikoagulation mit Citrat. In diesem Fall wird von einer dritten Pumpe durch eine Zweigleitung eine citrathaltige Infusionslösung, z.B. eine 4%-Na$_3$-Citratlösung, in das Blut in der Hauptleitung gefördert. Da diese Infusionslösung die Gerinnung des Blutes hemmt, befindet sich diese Zweigleitung in der Nähe der Blutentnahmestelle des extrakorporalen Kreislaufs, d.h. in der Nähe der arteriellen Nadel. Das Citrat komplexiert das im Blut enthaltene Calcium, wodurch die Gerinnungsfähigkeit des Blutes gehemmt wird. Ist in dem extrakorporalen Blutkreislauf ein Dialysator mit einer semipermeablen Membran vorhanden, wird dem Blut über diese Membran das Citrat mitsamt dem gebunden Calcium entzogen.

Da das Calcium im Blut komplexiert wird und bei Dialyseverfahren zusätzlich im Dialysator dem Blut das an Citrat gebundene Calcium entzogen wird, muss vor der Rückgabe des Blutes an den Patienten die Konzentration an freiem Calcium (nicht komplexiert) wieder in einen physiologischen Bereich gebracht werden. Zu niedrige Calciumkonzentrationen können zu Nebenwirkungen während der Behandlung führen. Zur Wiederherstellung der physiologischen Calciumkonzentration kann dem Blut über eine Zugabestelle, z.B. in Form eines T-Stücks, das im Blutkreislauf in der Nähe des venösen Patientenzugangs angeordnet ist, eine calciumhaltige Infusionslösung infundiert werden.

Hier ist die Anwendung des beschriebenen Verfahrens und der erfindungsgemäßen Vorrichtung besonders vorteilhaft, da eine schlechte Durchmischung von Blut mit der Calciumlösung zur Gerinnselbildung im T-Stück und Verschluss des extrakorporalen Kreislaufs führen kann.

In einer Ausführungsform des Verfahrens ist die Infusionslösung A eine calciumhaltige Lösung. In einer weiteren Ausführungsform des Verfahrens ist die Infusionslösung B eine citronensäurehaltige Infusionslösung.

In einer Ausführungsform des Verfahrens werden die im Verfahren verwendeten Pumpen von einer Kontrolleinheit gesteuert oder geregelt, so dass sie die im Verfahren angewendeten stetigen Flüsse oder Förderraten und die gepulsten Flussprofile bereitstellen.

In einer Ausführungsform des Verfahren werden zumindest eine Pumpe zur Förderung des Blutes im extrakorporalen Kreislauf und eine zweite Pumpe zur Förderung einer Infusionslösung A, z.B. einer calciumhaltigen Infusionslösung, von der Kontrolleinheit gesteuert oder geregelt.

In einer Ausführungsform des Verfahrens wird auch eine dritte Pumpe zur Förderung einer weiteren Infusionslösung B, z.B. einer Antikoagulanzlösung wie einer citronensäurehaltigen Infusionslösung, von der Kontrolleinheit gesteuert oder geregelt.

In einer Ausführungsform des Verfahren ist die Kontrolleinheit dazu konfiguriert, dass sie den Fluss der zweiten und/oder der dritten Pumpe in Abhängigkeit von der ersten Pumpe, die zur Förderung des Blutes im extrakorporalen Kreislauf dient, regelt oder steuert.

In einer Ausführungsform des Verfahrens kann die Kontrolleinheit dazu konfiguriert sein, dass sie bei Änderungen des Blutflusses automatisch den Fluss der zweiten und/oder der dritten Pumpe anpasst.

In einer Ausführungsform des Verfahrens ist die Kontrolleinheit dazu konfiguriert mittels eines hinterlegten Algorithmus aus dem mittleren Fluss (Vm) der Infusionslösung, der sich aus der gewünschten Dosierung ergibt, ein gepulstes Flussprofil, bestehend aus Pulsfluss (Vopt), einer Pulsweite (Tau2) und einer Pulsfrequenz $f$, zu berechnen, bei dem die Infusionslösung A mit einem Pulsfluss (Vopt) gefördert wird, der zu einer besseren Durchmischung der Infusionslösung A mit dem Blut führt.

Dieses gepulste Flussprofil kann automatisch angewendet werden oder über eine Benutzeroberfläche als Vorschlag angezeigt werden, welcher dann vom Anwender bestätigt werden muss.

In einer alternativen Ausführungsform des Verfahrens können alle oder einzelne Parameter des gepulsten Flussprofils, Pulsfluss (Vopt) und/oder Pulsweite (Tau2) und/oder Pulsfrequenz $f$ und/oder Fluss in der Pulspause über eine Benutzeroberfläche eingegeben und an die Kontrolleinheit weitervermittelt werden.

Bei einer Ausführungsform des Verfahren können der Kontrolleinheit diese Arbeitsparameter, d.h. die Flüsse oder Förderraten aller oder einzelner Pumpen, durch eine Benutzeroberfläche übermittelt werden. An dieser Benutzeroberfläche kann der Anwender die Arbeitsparameter, z.B. den Blutfluss, den Fluss des Antikoagulanzmittels und/oder den Fluss der zweiten Flüssigkeit, z.B. der calciumhaltigen Infusionslösung eingeben. Die Daten können dann an die Kontrolleinheit übermittelt werden, die dann die Pumpen dementsprechend regelt oder steuert.

Weiterhin ist die Möglichkeit gegeben, dass die Kontrolleinheit die während der Behandlung vorliegenden Arbeitsparameter durch Signale von den Pumpen erhält und dann z.B. den Fluss des Antikoagulanzmittels und/oder der zweiten Flüssigkeit, z.B. der calciumhaltigen Infusionslösung anpassen kann.

Bei einer Ausführungsform des Verfahrens kann dazu ein Teil der Arbeitsparameter auch durch eine Berechnungseinheit berechnet werden. Diese Berechnungseinheit kann Teil der Kontrolleinheit sein. Die Berechnungseinheit kann auch eine separat ausgeführte Vorrichtung sein, die mit der Kontrolleinheit verbunden ist. In der Berechnungseinheit kann z.B. ein Algorithmus hinterlegt sein, der nach Festlegung der gewünschten Blutflussrate oder Bestimmung der aktuell vorliegenden Blutflussrate der Fluss des Antikoagulanzmittels berechnet wird.

In einer Ausführungsform des Verfahrens kann die Berechnungseinheit aus den Werten für den Blutfluss und/oder der Antikoagulanzfluss auch den mittleren Volumenstrom der zweiten Flüssigkeit, z.B. der calciumhaltigen Infusionslösung berechnen. Es ist auch möglich, dass die Berechnungseinheit aus dem mittleren Volumenstrom der zweiten Flüssigkeit, z.B. der calciumhaltigen Infusionslösung, ein gepulstes Flussprofil berechnet. Die Berechnungseinheit bestimmt oder schlägt dann Werte für Pulsweite (Tau2), Pulsfrequenz $f$ und Pulsfluss (Vopt) vor. Dazu kann ein Algorithmus verwendet werden, der diese Werte so berechnet, dass sich der gewünschte mittlere Volumenstrom ergibt.

Alternativ können die Parameter Pulsweite (Tau2), Pulsfrequenz *f* und Pulsfluss (Vopt) vom Anwender festgelegt und in über die Benutzeroberfläche an die Kontrolleinheit und/oder Berechnungseinheit übermittelt werden.

Bei dem Verfahren kann das gepulste Flussprofil ein pfropfenförmiges Profil aufweisen.

Bei einem pfropfenförmigen Profil ist der Pulsfluss (Vopt) der Infusionlösung A größer als der Blutfluss, woraus sich sehr kurze Pulsweiten ergeben, da der mittlere Fluss Vm der Infusionslösung deutlich geringer ist als der Blutfluss.

In einer Aufführungsform beträgt der Pulsfluss (Vopt) das 1,1 bis 2 fache des Blutflusses, vorzugsweise das 1,3 bis 1,7 fache des Blutflusses.

Werte für den Blutfluss bei einer extrakorporalen Blutbehandlung können 100 bis 600 ml/min, bevorzugt 150-300ml/min betragen.

Werte für den mittleren Fluss der Infusionslösung (Vm) können 50-300 ml/h, bevorzugt 90-150 ml/h betragen.

Die Werte für die Pulsweite könnten dann für das pfropfenförmige Profil zwischen 10 und 100 ms, bevorzugt 20-50 ms betragen.

Die Werte für die Pulsfrequenz *f* ergeben sich dann aus Gleichung (1).

Bei kurzen Pulsweiten (z.B. >50ms) ist eine Kontrolle des Flusses nicht einfach. In einer Ausführungsform der Erfindung wird die Pumpe so angesteuert, dass sie ein bestimmtes Volumen pro Puls fördert. Die Kontrolle der Förderrate kann dann entfallen. Pulsfrequenz und/oder das Pulsvolumen werden variiert. Die mittlere Flussrate wird über Pulsfrequenz und Pulsvolumen gesteuert.

Alternativ kann der gepulste Fluss gekräuseltes Profil aufweisen. Hier liegen der Pulsfluss (Vopt) und der Blutfluss in der gleichen Größenordnung, typischerweise ist der Pulsfluss kleiner als der Blutfluss.

In einer Aufführungsform beträgt der Pulsfluss (Vopt) das 0,1 bis 1 fache des Blutflusses, vorzugsweise das 0,4-0,6 fache des Blutflusses.

Werte für den Blutfluss bei einer extrakorporalen Blutbehandlung können 100 bis 600 ml/min, bevorzugt 150-300ml/min betragen.

Werte für den mittleren Fluss der Infusionslösung (Vm) können 50-300 ml/h, bevorzugt 90-150 ml/h betragen.

Die Werte für die Pulsweite könnten dann für das gekräuselte Profil zwischen 0,1 und 5 s, bevorzugt 0,3-3 s betragen.

Die Werte für die Pulsfrequenz *f* ergeben sich dann aus Gleichung (1).

Die mittlere Flussrate wird hier über den Pulsfluss über Pulsfrequenz und/oder Pulsvolumen gesteuert.

In dem Verfahren können beliebige in der Medizintechnik bekannte Pumpen verwendet werden. Die zur Förderung des Blutes verwendete Pumpe muss speziell für das Pumpen von Blut geeignet sein. Für die Infusionslösungen A und B können beliebige, okkludierende Pumpen verwendet werden. In einer Ausführungsform handelt es sich um peristaltische Pumpen. In einer alternativen Ausführungsform handelt es sich Membranpumpen.

In einer Ausführungsform kann der extrakorporale Blutkreislauf des Verfahrens zur extrakorporalen Behandlung des Blutes eines Patienten dienen, vorzugsweise einer Dialysebehandlung. Eine Dialysebehandlung kann eine Hämodialyse-, eine Hämofiltration- und/oder eine Hämodiafiltrationsbehandlung sein.

Die Erfindung betrifft eine Vorrichtung zur extrakorporalen Blutbehandlung, umfassend zumindest eine erste Pumpe zur Förderung von Blut und eine zweite Pumpe zur Förderung einer Infusionslösung A sowie zumindest eine Kontrolleinheit, die die zweite Pumpe so regelt oder steuert, dass die zweite Pumpe die Infusionslösung A mit einem gepulsten Flussprofil fördert. Das gepulste Flussprofil wird gezielt durch die Steuersignale des Kontrolleinheit erzeugt.

Bei einem gepulsten Flussprofil wechseln sich Phasen, in denen die Flüssigkeit oder die Infusionslösung gefördert wird (Flusspulse oder Pulse) und Phasen, in denen keine oder deutlich weniger Flüssigkeit gefördert wird (Flusspausen), periodisch ab und ergeben so ein sogenanntes gepulstes Flussprofil. In anderen Worten, die zweite Pumpe läuft diskontinuierlich, was bedeutet, dass in der Förderrate der zweiten Pumpe prägnante Änderungen auftreten, die Pumpe wird nicht unbedingt in periodischen Abständen gestoppt.

Das gepulste Flussprofil kann dabei ein Rechteck-Profil aufweisen, ebenso geeignet ist aber auch ein sinusförmiges Profil ein nadelförmiges Profil (Delta oder Dirac-Funktion oder ähnliches).

Im Fall eines Rechteckprofils wird das gepulste Flussprofil definiert über die Pulsweite (Dauer der Pumpenbetriebs oder der Förderung der Flüssigkeit) Tau2, die Pulspause (Dauer des Pumpenstillstandes) Tau1, die sich daraus ergebende Pulsfrequenz *f* und der im Puls geförderte Flüssigkeitsmenge pro Zeit oder der Förderrate, den Pulsfluss (Vopt). Es ist auch denkbar, dass in der Pulspause die Pumpe nicht zum Stillstand kommt, sondern mit einer deutlich geringeren Förderrate weiterläuft. Dies müsste dann bei der Berechnung der mittleren Förderrate berücksichtigt werden. Aus den vorgenannten Parametern ergibt sich mittlere Fluss oder die mittlere Förderrate (Vm) der Flüssigkeit oder der Infusionslösung A über ein längeres Zeitintervall.

In einer Ausführungsform der erfindungsgemäßen Vorrichtung steuert oder regelt die Kontrolleinheit die erste Pumpe, die das Blut fördert, so dass die erste Pumpe kontinuierlich läuft um das Blut mit einem stetigen Fluss zu fördern.

Für den Fachmann selbstverständlich ist, dass während einer Behandlung durch äußere Umstände Flussänderungen auftreten oder notwendig werden können, die aber die prinzipielle Funktionsweise des Verfahrens nicht beeinträchtigen.

Für den Fachmann selbstverständlich ist ebenso, dass bedingt durch die Struktur der Pumpen, Druck- und Flusspulse entstehen können. Dieses von der Pumpenstruktur erzeugte Flussprofil ist von der Erfindung nicht umfasst.

Die Vorrichtung ist zur Durchführung einer extrakorporalen Blutbehandlung mit einer regionalen Antikoagulation geeignet. Die Vorrichtung weist eine weitere, dritte Pumpe zur Förderung einer weiteren Flüssigkeit, einer Infusionslösung B, vorzugsweise eines Antikoagulanzmittels. Für diese Ausführungsform wird die dritte Pumpe dann als Antikoagulanzpumpe bezeichnet. Dieses Antikoagulanzmittel ist vorzugsweise eine Citronensäure- und/oder Citrat-haltige Lösung. Bei der regionalen Antikoagulation mit einer Citronensäure- und/oder Citrathaltigen Lösung ist die Infusionslösung A, die zur Neutralisierung des Antikoagulanzmittels dient vorzugsweise eine calciumhaltige Infusionslösung. In dieser Ausführungsform wird die zweite Pumpe als Calciumpumpe bezeichnet.

In einer Ausführungsform wird die weitere, dritte Pumpe, bevorzugt die Antikoagulanzpumpe, ebenfalls durch die Kontrolleinheit gesteuert oder geregelt. Die weitere, dritte Pumpe fördert das Antikoagulanzmittel vorzugsweise kontinuierlich mit einem stetigen Fluss. Alternativ kann das Antikoagulanzmittel auch mit einem gepulsten Flussprofil gefördert werden.

Bei der regionalen Antikoagulation des extrakorporalen Kreislaufs ist es vorteilhaft die Förderraten der drei verwendeten Pumpen, Blutpumpe, die Antikoagulanzpumpe und die Calciumpumpe aufeinander abzustimmen.

In einer Ausführungsform ist die Kontrolleinheit dazu ausgelegt, bei der Regelung oder Steuerung der zweiten Pumpe, bevorzugt der Calciumpumpe, und der dritten Pumpe, bevorzugt der Antikoagulanzpumpe, die Arbeitsparameter oder Förderrate der ersten Pumpe, der Blutpumpe, zu berücksichtigen.

Bei der erfindungsgemäßen Vorrichtung ist die Kontrolleinheit dazu konfiguriert mittels eines hinterlegten Algorithmus z.B aus dem mittleren Fluss (Vm) der Infusionslösung, der sich aus der gewünschten Dosierung ergibt, ein gepulstes Flussprofil, bestehend z.B. aus Pulsfluss (Vopt), einer Pulsweite (Tau2) und einer Pulsfrequenz *f*, zu berechnen, bei dem die Infusionslösung A mit einem Pulsfluss (Vopt) gefördert wird, der zu einer besseren Durchmischung der Infusionslösung A mit dem Blut führt.

Dieses gepulste Flussprofil kann automatisch angewendet werden oder über eine Benutzeroberfläche als Vorschlag angezeigt werden, welcher dann vom Anwender bestätigt werden muss.

In einer alternativen Ausführungsform der erfindungsgemäßen Vorrichtung kann eine Benutzeroberfläche vorgesehen sein, über die alle oder einzelne Parameter des gepulsten Flussprofils, Pulsfluss (Vopt) und/oder Pulsweite (Tau2) und/oder Pulsfrequenz *f* und/oder Fluss in der Pulspause und die Arbeitsparameter, d.h. die Flüsse oder Förderraten aller oder einzelner Pumpen, eingegeben und an die Kontrolleinheit weitervermittelt werden können.

An dieser Benutzeroberfläche kann der Anwender die Arbeitsparameter, z.B. den Blutfluss, den Fluss des Antikoagulanzmittels und/oder den Fluss der zweiten Flüssigkeit, z.B. der calciumhaltigen Infusionslösung eingeben. Die Daten können dann an die Kontrolleinheit übermittelt werden, die dann die Pumpen dementsprechend regelt oder steuert.

Weiterhin ist die Möglichkeit gegeben, dass die Kontrolleinheit die während der Behandlung vorliegenden Arbeitsparameter durch Signale von den Pumpen erhält und dann z.B. den Fluss des Antikoagulanzmittels und/oder der zweiten Flüssigkeit, z.B. der calciumhaltigen Infusionslösung anpassen kann.

Bei einer Ausführungsform der erfindungsgemäßen Vorrichtung kann zumindest ein Teil der Arbeitsparameter der Pumpen und/oder der Parameter des gepulsten Flussprofils auch durch eine Berechnungseinheit berechnet werden. Diese Berechnungseinheit kann Teil der Kontrolleinheit sein. Die Berechnungseinheit kann auch eine separat ausgeführte Vorrichtung sein, die mit der Kontrolleinheit verbunden ist. In der Berechnungseinheit kann z.B. ein Algorithmus hinterlegt sein, der nach Festlegung der gewünschten Blutflussrate oder Bestimmung der aktuell vorliegenden Blutflussrate der Fluss des Antikoagulanzmittels berechnet wird.

In einer Ausführungsform der erfindungsgemäßen Vorrichtung kann die Berechnungseinheit aus den Werten für den Blutfluss und/oder der Antikoagulanzfluss auch den mittleren Volumenstrom der zweiten Flüssigkeit, z.B. der calciumhaltigen Infusionslösung berechnen. Es ist auch möglich, dass die Berechnungseinheit aus dem mittleren Volumenstrom der zweiten Flüssigkeit, z.B. der calciumhaltigen Infusionslösung, ein gepulstes Flussprofil berechnet. Die Berechnungseinheit bestimmt oder schlägt dann Werte für Pulsweite (Tau2), Pulsfrequenz *f* und Pulsfluss (Vopt) vor. Dazu kann ein Algorithmus verwendet werden, der diese Werte so berechnet, dass sich der gewünschte mittlere Volumenstrom ergibt.

Alternativ können die Parameter Pulsweite (Tau2), Pulsfrequenz *f* und Pulsfluss (Vopt) vom Anwender festgelegt und in über die Benutzeroberfläche an die Kontrolleinheit und/oder Berechnungseinheit übermittelt werden.

Die erfindungsgemäße Vorrichtung kann in einer Ausführungsform ein gepulstes Flussprofil als ein pfropfenförmiges Profil erzeugen.

Bei einem pfropfenförmigen Profil ist der Pulsfluss (Vopt) der Infusionlösung A größer als der Blutfluss, woraus sich sehr kurze Pulsweiten ergeben, da der mittlere Fluss Vm der Infusionslösung deutlich geringer ist als der Blutfluss.

In einer Auführungsform beträgt der Pulsfluss (Vopt) das 1,1 bis 2 fache des Blutflusses, vorzugsweise das 1,3 bis 1,7 fache des Blutflusses.

Werte für den Blutfluss bei einer extrakorporalen Blutbehandlung können 100 bis 600 ml/min, bevorzugt 150-300ml/min betragen.

Werte für den mittleren Fluss der Infusionslösung (Vm) können 50-300 ml/h, bevorzugt 90-150 ml/h betragen.

Die Werte für die Pulsweite könnten dann für das pfropfenförmige Profil zwischen 10 und 100 ms, bevorzugt 20-50 ms betragen.

Die Werte für die Pulsfrequenz $f$ ergeben sich dann aus Gleichung (1).

Bei kurzen Pulsweiten (z.B. >50ms) ist eine Kontrolle des Flusses nicht einfach. In einer Ausführungsform der Erfindung wird die Pumpe so angesteuert, dass sie ein bestimmtes Volumen pro Puls fördert. Die Kontrolle der Förderrate kann dann entfallen. Pulsfrequenz und/oder das Pulsvolumen werden variiert. Die mittlere Flussrate wird über Pulsfrequenz und Pulsvolumen gesteuert.

Alternativ kann in einer Ausführungsform der durch die erfindungsgemäße Vorrichtung erzeugte, gepulste Fluss ein gekräuseltes Profil aufweisen. Hier liegen der Pulsfluss (Vopt) und der Blutfluss in der gleichen Größenordnung, typischerweise ist der Pulsfluss kleiner als der Blutfluss.

In einer Aufführungsform beträgt der Pulsfluss (Vopt) das 0,1 bis 1 fache des Blutflusses, vorzugsweise das 0,4-0,6 fache des Blutflusses.

Werte für den Blutfluss bei einer extrakorporalen Blutbehandlung können 100 bis 600 ml/min, bevorzugt 150-300ml/min betragen.

Werte für den mittleren Fluss der Infusionslösung (Vm) können 50-300 ml/h, bevorzugt 90-150 ml/h betragen.

Die Werte für die Pulsweite könnten dann für das gekräuselte Profil zwischen 0,1 und 5 s, bevorzugt 0,3-3 s betragen.

Die Werte für die Pulsfrequenz $f$ ergeben sich dann aus Gleichung (1).

Die mittlere Flussrate wird hier über den Pulsfluss über Pulsfrequenz und/oder Pulsvolumen gesteuert.

Das von der Berechnungseinheit erstellte Flussprofil kann dem Anwender über eine Benutzeroberfläche angezeigt werden und erst nach Bestätigung durch diesen ausgeführt werden.

In einer Ausführungsform der Vorrichtung kann die zweite Pumpe eine peristaltische Pumpe.

In einer alternativen Ausführungsform der Vorrichtung kann die zweite Pumpe eine Membranpumpe ist.

In einer Ausführungsform kann die Vorrichtung zur extrakorporalen Blutbehandlung z.B. eine Maschine für die Hämodialyse, die Hämodiafiltration und/oder die Hämodialfiltration sein. Die Vorrichtung kann zur Durchführung einer oder mehrerer dieser Behandlungsarten geeignet sein.

## Kurze Beschreibung der Zeichnungen

Weitere Einzelheiten und Vorteile der Erfindung werden anhand der in den Zeichnungen dargestellten Ausführungsbeispiele näher beschrieben. Sie zeigen:

Figur 1: Schematische Darstellung eines extrakorporalen Kreislaufs mit regionaler Antikoagulation und Kontrolleinheit zur Ausführung des Verfahrens
Figur 2: Darstellung eines gepulsten Flussprofils
Figur 3: Darstellung eines gepulsten Flussprofils mit pfropfenförmigen Pulsen

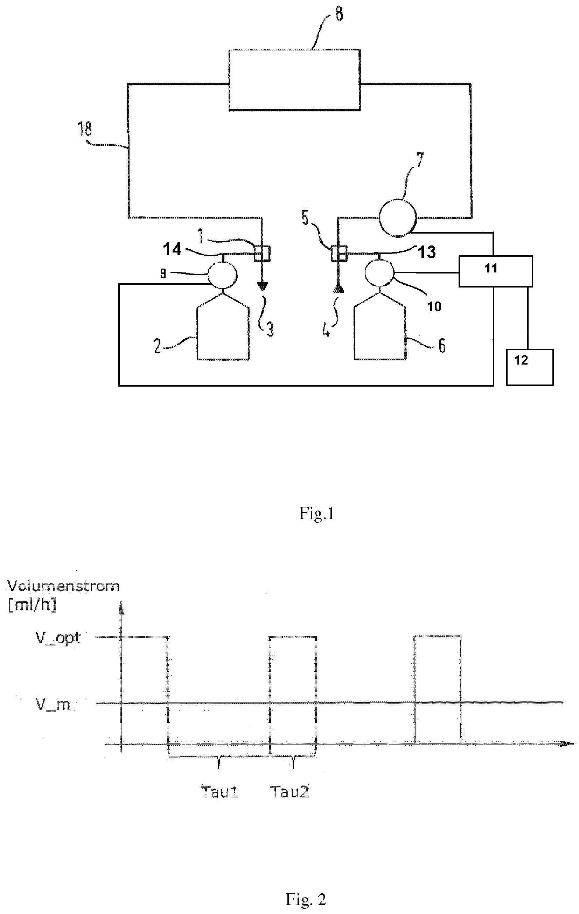

In Figur 1 ist schematisch ein Blutkreislauf gezeigt, der zur Durchführung des Verfahrens geeignet ist.

Vom arteriellen Patientenzugang 4 wird das Blut von der Blutpumpe 7 durch die Hauptleitung 18 des Schlauchsystems des extrakorporalen Kreislaufs mit dem Dialysator 8 gefördert. Vor der Blutpumpe 7 ist in der Hauptleitung 18 des extrakorporalen Kreislaufs eine erste Zugabestelle 5 in Form eines handelsüblichen T-Stücks vorgesehen. An dieser ersten Zugabestelle 5 wird über die Zweigleitung 13 durch eine weitere Pumpe 10 ein Antikoagulanzmittel 6, hier dargestellt in Form eines Citratbeutels, der z.B. 4%-iges $Na_3$-Citrat enthält, in die Hauptleitung 18 des extrakorporalen Blutkreislaufs infundiert. In unmittelbarer Nähe der venösen Patientenzugangs 3 befindet sich eine zweite Zugabestelle 1 in Form eines weiteren handelsüblichen T-Stücks, an der dem Blut über eine Zweigleitung 14 durch die zweite Pumpe 9 eine Infusionslösung A 2, hier in Form einer Calciumlösung, zugegeben wird.

Über die Benutzeroberfläche 12 kann der Anwender Behandlungsparameter eingeben, unter anderem den gewünschten Blutfluss, den gewünschten Citratfluss und die gewünschte Calciumdosierung ein. Der Benutzer kann verschiedene Optionen für das gepulste Flussprofil der Pumpen 7, 9 und 10 wählen. Die Benutzeroberfläche 12 kann auch zur Anzeige von Daten für den Benutzer dienen.

Zwischen der Benutzeroberfläche 12 und der Kontrolleinheit 11 werden die Daten übermittelt. Die Kontrolleinheit 11 steuert oder regelt dann die Pumpen 7, 9 und 10. Die Kontrolleinheit 11 kann aus den Daten für den Blutfluss, die Citratdosierung und die Calciumdosierung errechnen. Aus der vom Anwender angegebenen Calciumdosierung wird dann von einer Berechnungseinheit (nicht gezeigt) ein gepulstes Flussprofil berechnet. Bei Änderungen eines Parameters während der Behandlung kann eine Neuberechnung des gepulsten Flussprofils erfolgen.

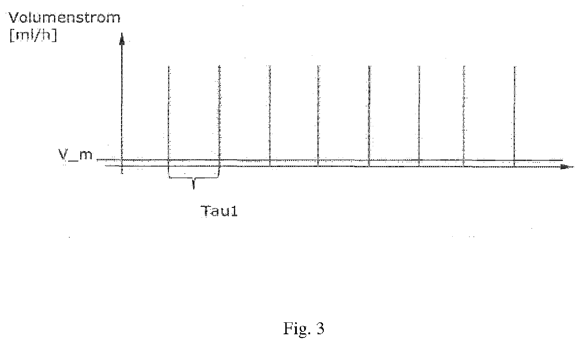

Die Kontrolleinheit 11 steuert die Blutpumpe 7 so, dass das Blut kontinuierlich mit einem vorgegebenen, stetigen Fluss gefördert wird. Abhängig vom Patienten oder Filter können während der Behandlung Anpassungen nötig sein. Die Antikoagulanzlösung 6 kann wahlweise kontinuierlich oder mit einem gepulsten Flussprofil gefördert werden. Die Kontrolleinheit steuert die Pumpe 9 für die Infusionslösung A 2 so an, dass diese mit einem gepulsten Flussprofil gefördert wird. Exemplarische Beispiele für solche gepulsten Flussprofile sind in Figur 2 und 3 gezeigt.

Figur 2 zeigt ein gekräuseltes Flussprofil, bei dem die Pulsweiten (Tau2) eine Dauer von 300ms aufweisen.

Figur 3 zeigt ein gekräuseltes Flussprofil, wo die Pulsweite der Förderphasen der Pumpe sehr kurz sind. Dies wird durch eine höhere Frequenz *f* und einem höheren Pulsfluss ausgeglichen. Hierbei entsteht eine Pfropfenströmung im Blut. Der Pulsfluss liegt hier im Bereich des Flusses der Blutpumpe und höher, z.B. 100 oder 500 ml/min.

Die Frequenz liegt im Bereich von 20 bis 60/min, kann bei entsprechend schneller Ansteuerung jedoch auch deutlich höher liegen.

Das Verfahren gewährleistet eine optimierte Mischung zweier Lösungen in einem Schlauchsystem. Dies ist besonders vorteilhaft bei dem Verfahren der regionalen Antikoagulation mit Citronensäure, wo es an der Zugabestelle der calciumhaltigen Infusionslösung zur Gerinnselbildung kommen kann. Mit dem Verfahren und der erfindungsgemäßen Vorrichtung sind durch das spezielle Flussprofil der zugegebenen calciumhaltigen Infusionslösung keine speziell ausgeführten Zugabestellen nötig. Die optimierte Mischung wird von der Behandlungsmaschine selbst gewährsleistet. Es müssen keine speziellen Blutschlauchsysteme angewendet werden. Das Verfahren und die Vorrichtung sind aber auch sonst überall dort vorteilhaft, wo man eine effektive Mischung zweier Lösungen anstrebt.

## Patentansprüche

1. Vorrichtung zur extrakorporalen Blutbehandlung umfassend zumindest eine erste Pumpe (7) zur Förderung von Blut, zumindest eine zweite Pumpe (9) zur Förderung einer Infusionslösung A (2), eine dritte Pumpe (10) zur Förderung einer weiteren Infusionslösung in Form eines Antikoagulanzmittels und zumindest eine Kontrolleinheit (11), die (11) die zweite Pumpe (9) so steuert oder regelt, dass die zweite Pumpe die Infusionslösung A (2) mit einem gepulsten Flussprofil fördert, **gekennzeichnet dadurch, dass** die Kontrolleinheit (11) mittels eines hinterlegten Algorithmus unter Berücksichtigung des Flusses der ersten Pumpe (7) und/ oder der dritten Pumpe (10) ein gepulstes Flussprofil für die zweiten Pumpe (9) berechnet und das gepulste Flussprofil gezielt durch die Steuersignale der Kontrolleinheit erzeugt wird.

2. Vorrichtung nach Anspruch 1 wobei durch die Kontrolleinheit (11) die erste Pumpe (7) so gesteuert oder geregelt wird, dass diese kontinuierlich läuft um das Blut mit einem stetigen Fluss zu fördern.

3. Vorrichtung nach Anspruch 1 oder 2, **gekennzeichnet dadurch, dass** die Kontrolleinheit (11) die dritte Pumpe (10) steuert oder regelt.

4. Vorrichtung nach einem der Ansprüche 1 bis 3, **gekennzeichnet dadurch, dass** die Vorrichtung eine Benutzeroberfläche (12) zur Eingabe der Arbeitsparameter der ersten Pumpe (7) und oder der zweiten Pumpe (9) und/oder der weiteren, dritten Pumpe (10) aufweist und diese Benutzeroberfläche die Arbeitsparameter an die Kontrolleinheit übermittelt.

5. Vorrichtung nach einem der Ansprüche 1 bis 4, **gekennzeichnet dadurch, dass** es sich bei der Vorrichtung um eine Maschine zur Blutbehandlung, vorzugsweise eine Dialysemaschine handelt, z.B. in Form einer Hämodialysemaschine, einer Hämofiltrationsmaschine und/oder einer Hämodiafiltrationsmaschine.

## Claims

1. A device for extracorporeal treatment of blood comprising at least one first pump (7) for conveying blood, at least one second pump (9) for conveying an infusion solution A (2), a third pump (10) for conveying an additional infusion solution in the form of an anticoagulant, and at least one control unit (11), said control unit (11) controlling or regulating the second pump (9), so that the second pump conveys the infusion solution A (2) with a pulsed flow profile, **characterized in that** the control unit (11) calculates a pulsed flow profile for the second pump (9) by means of an algorithm saved in the control unit, taking into account the flow of the first pump (7) and/or of the third pump (10), and the pulsed flow profile is generated in a targeted manner by the control signals of the control unit.

2. The device according to claim 2, wherein the control unit (11) controls or regulates the first pump (7), so that it runs continuously to convey the blood at a steady flow.

3. The device according to claim 1 or 2, **characterized in that** the control unit (11) controls or regulates the third pump (10).

4. The device according to any one of claims 1 to 3, **characterized in that** the device has a user interface (12) for input of the working parameters of the first pump (7) and/or of the second pump (9) and/or of the additional third pump (10), and this user interface transmits the working parameters to the control unit.

5. The device according to any one of claims 1 to 4, **characterized in that** the device is a machine for treatment of blood, preferably a dialysis machine, for example, in the form of a hemodialysis machine, a hemofiltration machine and/or a hemodiafiltration machine.

**Revendications**

1. Dispositif de traitement sanguin extracorporel, comprenant au moins une première pompe (7) pour le transport de sang, au moins une deuxième pompe (9) pour le transport d'une solution de perfusion A (2), une troisième pompe (10) pour le transport d'une autre solution de perfusion sous forme d'un produit anticoagulant et au moins une unité de commande (11), qui (11) commande et règle la deuxième pompe (9) de manière à ce que la deuxième pompe transporte la solution de perfusion A (2) avec un profil d'écoulement pulsé, **caractérisé en ce que** l'unité de commande (11) calcule, au moyen d'un algorithme enregistré, en tenant compte de l'écoulement de la première pompe (7) et/ou de la troisième pompe (10), un profil d'écoulement pulsé pour la deuxième pompe (9) et que le profil d'écoulement pulsé est généré de manière ciblée par les signaux de commande de l'unité de commande.

2. Dispositif selon la revendication 1, dans lequel la première pompe (7) est commandée ou réglée par l'unité de commande (11) de manière à fonctionner en continu pour transporter le sang avec un flux constant.

3. Dispositif selon la revendication 1 ou 2, **caractérisé en ce que** l'unité de commande (11) commande ou règle la troisième pompe (10).

4. Dispositif selon une des revendications 1 à 3, **caractérisé en ce que** le dispositif présente une surface d'utilisateur (12) pour la saisie des paramètres de travail de la première pompe (7) et/ou de la deuxième pompe (9) et/ou de la troisième pompe (10) et que cette surface d'utilisateur transmet les paramètres de travail à l'unité de commande.

5. Dispositif selon une des revendications 1 à 4, **caractérisé en ce que** le dispositif est une machine de traitement sanguin, de préférence une machine de dialyse, par exemple sous la forme d'une machine d'hémodialyse, d'une machine d'hémofiltration et/ou d'une machine d'hémodiafiltration.

Fig.1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2009030973 A
- US 8123396 B
- WO 2010029401 A1
- US 20070062861 A
- WO 2012104072 A1